# EUROPEAN PATENT SPECIFICATION

(11) **EP 2 352 528 B1**
(45) Date of publication and mention of the grant of the patent: **14.08.2019**
(21) Application number: 09760658.6
(22) Date of filing: 11.11.2009
(51) Int. Cl.: A61L 15/48, C11D 1/74

(54) **SUBSTRATE WITH ADHERENCE FOR FECES AND MENSES**
SUBSTRAT MIT HAFTUNG FÜR FÄZES UND MENSES
SUBSTRAT POSSÉDANT UNE ADHÉRENCE POUR LES SELLES ET LES MENSTRUATIONS

(30) Priority: 14.11.2008 US 114496 P
(43) Date of publication of application: 10.08.2011
(73) Proprietor: The Procter & Gamble Company, Cincinnati, OH 45202 (US)
(72) Inventor: VEGA, Victor, Nicholas, Cincinnati Ohio 45231 (US); QIN, Wendy, West Chester Ohio 45069 (US); MARSH, Randall, Glann, Hamilton Ohio 45011 (US); KLOFTA, Thomas, James, Cincinnati Ohio 45249 (US)
(74) Representative: Heide, Ute
(86) International application number: PCT/US2009/063940
(87) International publication number: WO 2010/056685

(56) References cited:
- GB-A- 2 394 898
- US-A1- 2003 167 043

## Description

### FIELD OF THE INVENTION

A composition applied on the body facing surface of an absorbent article such as a diaper, training pant, adult incontinence product, feminine hygiene product, may be used to impart the adherence of feces or menses to the article. Such composition makes the cleaning task easier.

### BACKGROUND OF THE INVENTION

Many types of disposable absorbent products, such as diapers and sanitary napkins, having a high capacity for absorbing fluids such as urine or menses, are available. Those disposable absorbent products may have a topsheet comprising a lotion to deliver skin benefits to the skin of the wearer. Examples of diapers having a lotioned topsheet are disclosed in WO 96/16682. In recent years, the focus has been to deliver lotions to sanitary napkins and diapers that provide extra skin benefits, for example by addition of botanical ingredients or pharmaceutical ingredients to the lotion.

The addition of lotion to the topsheet of absorbent articles is also known in some cases to provide benefits such as improving the removal of feces or menses from the skin. The topsheet transfers a lotion to the skin that comprises ingredients limiting the adherence of menses or feces to the skin. However, the feces or menses still remain on the skin until it is cleaned off by the care taker or user.

U.K. Patent Application GB 2 394 898 A refers to an eyelid margin wipe comprising chemical means for adjusting the temperature of the wipe relative to the ambient temperature. The wipe is useful for treatment of disorders of the eyelid or eyelid margin.

U.S. Patent Application No. US 2003/0167043 A1 is directed to an absorbent article containing a liquid pervious topsheet coated with a lotion composition. The lotion composition comprises a plastic or fluid emollient such as mineral oil or petrolatum, an immobilizing agent such as a fatty alcohol or paraffin wax to immobilize the emollient on the surface of the topsheet, and optionally a hydrophilic surfactant to improve wettability of the coated topsheet.

The inventors have found that it would be also advantageous to develop diapers having a topsheet exhibiting adherence for bodily exudates, such as feces, thereby improving the ease of the cleaning task. More generally, it would be advantageous to develop disposable absorbent articles having a substrate or a sheet comprising a compound or a composition that exhibits adherence for bodily exudates such as feces or menses, so that less bodily exudates (feces or menses) remain on the skin of the user. The compound(s) or compositions thereof, imparting adherence for bodily exudates could also advantageously improve the cleaning task when they are incorporated in other skin-contacting materials such as tissues or wet wipes, to facilitate adherence of such bodily exudates to said tissue or wipe.

It is hence an object of the present invention to provide a substrate comprising a compound or composition imparting adherence for feces or menses to the substrate.

### SUMMARY OF THE INVENTION

The present invention relates to a personal care product as defined in claim 1. Preferred aspects of the invention are defined in dependent claims.

The present invention is also dedicated to a method for imparting adherence for body exudates to a personal care product as defined in claim 9.

### DETAILED DESCRIPTION OF THE INVENTION

As used herein "absorbent article" refers to devices which are intended to be placed against the skin of a wearer to absorb and contain the various exudates discharged from the body. Examples of absorbent articles include incontinence articles such as infant or adult diapers; pant-like diapers such as training pants; diaper holders; incontinence briefs. Further examples of absorbent articles are feminine hygiene products such as sanitary napkins and panti-liners. In a preferred embodiment of the present invention, the absorbent articles are diapers, pant-like diapers, sanitary napkins and panty-liners.

"Exudates" as used herein refers to material from a source internal to the body, such as menses, feces and mucus.

### SUBSTRATE USEFUL IN THE PRESENT INVENTION

The term "substrate" as used herein generally refers to a material suitable for manufacturing of, or suitable as, or in a topsheet, or a leg cuff, or a barrier cuff or a side flap or a wing of an absorbent article; or a material suitable for manufacturing of, or suitable as, or in a wipe. Suitable materials include woven and nonwoven materials (typically web materials, including woven or nonwoven webs of fibers); polymeric web materials such as (apertured formed) thermoplastic films, (apertured) plastic films, hydroformed thermoplastic films, porous foams, reticulated foams, reticulated thermoplastic films and thermoplastic scrims; paper tissue or combinations thereof.

"Nonwoven material" as used herein refers to a manufactured web of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled.

Preferred substrates herein may be or may comprise a nonwoven web material, whereby said nonwoven web may be manufactured by a wide number of known techniques. Non-limiting examples of techniques include spunbonding, carding, wet-laid, air-laid, melt-blown, needle-punching, mechanical entangling, thermo-mechanical entangling, hydroentangling, calender bonding and combination thereof.

Suitable substrates include material such as web material (e.g, woven or nonwoven web) comprising natural fibers or synthetic fibers or combination thereof. Examples of natural fibers may include cellulosic natural fibers, such as fibers from hardwood sources, softwood sources, or other non-wood plants. The natural fibers may comprise cellulose, starch and combinations thereof. The synthetic fibers can be any material, such as, but not limited to, those selected from the group consisting of polyesters (e.g., polyethylene tcrcphthalatc), polyolcfins, polypropylcncs, polyethylenes, polyethers, polyamides, polyesteramides, polyvinylalcohols, polyhydroxyalkanoates, polysaccharides, and combinations thereof. Further, the synthetic fibers can be a single component (i.e., single synthetic material or mixture makes up entire fiber), bi-component (i.e., the fiber is divided into regions, the regions including two or more different synthetic materials or mixtures thereof and may include co-extruded fibers and core and sheath fibers) and combinations thereof. Bi-component fibers can be used as a component fiber of the web material, and/or they may be present to act as a binder for the other fibers present in the web material. Any or all of the synthetic fibers may be treated before, during, or after manufacture to change any desired properties of the fibers.

The substrate may also be dispersible, that is, the substrate may sufficiently dissolve or disintegrate in water such that the substrate may be discarded in sewer or septic systems without presenting any problems for typical household or municipal sanitization systems. The materials and methods for making such a dispersible substrate are described, for example, in WO 2007/125443 to Kimberly-Clark Worldwide, Inc.; in U.S. Patent No. 4,755,421 to Manning et al.; in U.S. Patent No. 7,285,504 to Jones et al.; in U.S. Patent No. 7,157,389 to Branham et al.; and in U.S. Patent No. 7,101,612 to Lang et al.

Suitable substrates also include paper substrates comprising paper tissue or paper towel technology in general, including but not limited to conventionally felt-pressed or conventional wet pressed tissue paper; pattern densified tissue paper; and high-bulk, uncompacted tissue paper. Non-limiting examples of tissue-towel paper products include toweling, facial tissue, bath tissue, table napkins and the like. Useful papermaking fibers include cellulosic fibers commonly known as wood pulp fibers. Applicable wood pulps include chemical pulps, such as Kraft, sulfite, and sulfate pulps, as well as mechanical pulps including, for example, groundwood, thermomechanical pulp and chemically modified thermomechanical pulp. Pulps derived from both deciduous trees (hereinafter, also referred to as "hardwood") and coniferous trees (hereinafter, also referred to as "softwood") may be utilized. The hardwood and softwood fibers can be blended, or alternatively, can be deposited in layers to provide a stratified web.

The substrate useful in the present invention may be comprised by, or may form, or may be used for manufacturing of, the topsheet of an absorbent article. The topsheet is oriented towards and contacts the body of the wearer permitting bodily discharges to rapidly penetrate through it without allowing fluid to flow back through the topsheet to the skin of the wearer.

A suitable substrate for use in, or as such a topsheet may be manufactured from a wide range of materials such as woven and nonwoven materials; polymeric materials such as thermoplastic films, apertured formed thermoplastic films, plastic films, apertured plastic films, and hydroformed thermoplastic films; porous foams; reticulated foams; reticulated thermoplastic films; and thermoplastic scrims; preferred substrates are nonwoven materials and apertured formed thermoplastic films. The topsheet is preferably made of a hydrophobic material to isolate the wearer's skin from liquids which have passed through the topsheet and are contained in the absorbent core (i.e., to prevent rewet).

The substrate comprised by, or forming, or used for manufacturing of, the topsheet of an absorbent article may alternatively, or additionally be apertured, i.e the topsheet has a plurality of apertures having an aperture size of at least about 0.2 mm². The topsheet has preferably an open area of at least about 10%, the open area being the sum of all apertures. The open are may be determined by the procedure disclosed in WO 95/05139.

The substrate may be comprised by, or may form, or may be used for manufacturing of a topsheet that has one or more openings. Typically, the openings are large enough to let feces or menses pass to a void space underneath said topsheet, also referred to as anal cuff or vaginal cuff. For example, U.S. Patent Application No. 2006/0058766 A, filed on September 13, 2005 discloses an absorbent article wherein the topsheet is provided with at least one opening adapted to receive fecal material. Such topsheets may be made of or may comprise a liquid impervious material, and thus, the subtrate may be a liquid impervious material.

The substrate useful in the present invention may also be comprised by, or may form, or may be used for manufacturing of the legs and/or the barrier cuffs and/or the wings and/or the side flaps of an absorbent article. Any of the above-described nonwoven web material may for example be used as or in such cuffs.

The substrate useful in the present invention may be or may comprise a laminate web of two or more nonwoven webs. The laminate web may comprise spunbond layer(s) (S), and/or meltblown laycr(s), and/or carded laycr(s). Suitable laminate webs include, but arc not limited to, SS, SSS, SMS or SMMS. Suitable examples of nonwoven laminate substrates, in particular when used as cuffs, side flaps or wings, include, for example, 34 gsm SMMS (with 12 gsm for each meltblown and 5 gsm for each spunbond layer); 34 gsm SMMS (with 10 gsm for each meltblown and 7 gsm for each spunbond layer); 30 gsm SMMS (with 10 gsm for each meltblown and 5 gsm for each spunbond layer); 30 gsm SMMS (with 8 gsm for each meltblown and 7 gsm for each spunbond layer); 34 gsm SMS (with 20 gsm for the meltblown and 7 gsm for each spunbond layer), or, for example, laminate webs comprising two webs of 17 gsm SMMS laminated to each other.

The substrate useful in the present invention may have a basis weight between about 5 to 100g/m². Where the substrate is comprised by, or forms, or is used for manufacturing of the topsheet, the legs and/or the barrier cuffs and/or the side flaps and/or the wings of an absorbent article it may have for example a basis weight between about 5 to 100 g/m², or between about 10 to 40 g/m², or between about 10 to 30 g/m².

Another suitable substrate may be Fibrella™ 3100 which is a 62 g/m² nonwoven web comprising 50% 1.5 denier polypropylene fibers and 50% 1.5 denier viscose fibers by weight of the substrate. In both of these commercially available fibrous webs, the average fiber length is about 38 mm. Another suitable material for use as a substrate may be SAWATEX™ 2642 as available from Sandler AG of Schwarzenbach/Salle, Germany. Yet another suitable material for use as a substrate may have a basis weight of from about 50 g/m² to about 60 g/m² and have a 20/80 blend of viscose fibers and polypropylene fibers. The substrate may also be a 60/40 blend of pulp and viscose fibers by weight of the substrate. Various examples of suitable substrates are disclosed in the patent literature, see for example U.S. Pat. No.6,960,349 B2, col.10 line 24 to col.11 line 39.

### COMPOUND(S) AND COMPOSITIONS USEFUL IN THE PRESENT INVENTION

### THE SOIL ADHERING COMPOUND

The substrate comprised by the personal care product of the present invention comprises at least a solid nonionic compound of the formula (referred in the present disclosure as soil adhering compound):

R₁-CO-[A]-R₂

wherein R₁ is a C₁₂-C₂₂ hydrocarbyl or C₁₂-C₂₂ substituted hydrocarbyl group;
wherein R₂ is selected from:
   (a) -O-CO-R₃ wherein R₃ is a C₁₂-C₂₂ hydrocarbyl or C₁₂-C₂₂ substituted hydrocarbyl group; or
   (b) 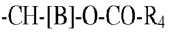 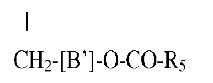
      wherein R₄ is a C₁₂-C₂₂ hydrocarbyl or C₁₂-C₂₂ substituted hydrocarbyl group;
      wherein R₅ is a C₁₂-C₂₂ hydrocarbyl or C₁₂-C₂₂ substituted hydrocarbyl group;
wherein A, B and B' are independently selected from: a polymer or copolymer of ethylene oxide, propylene oxide, butylene oxide, hydroxypropylcnc oxide wherein said polymer or said copolymer comprises from 2 to 200 monomers.

In the above formula, A, B and B' may be independently selected from polymers of ethylene oxide, propylene oxide, butylene oxide or from copolymers of ethylene oxide, propylene oxide, butylene oxide, hydroxypropylene oxide wherein said polymers or copolymers comprise from 2 to 200 monomers. Copolymers of ethylene oxide and/or propylene oxide and/or butylene oxide and/or hydroxypropylene oxide include alternating copolymers wherein the monomers are arranged in a regular alternating sequence, periodic copolymers wherein the monomers are arranged in a repeating sequence, random copolymers with random sequences of monomers and block copolymers comprising two or more homopolymer subunits linked by covalent bonds. In one embodiment, A, B and B' may be identical.

In the above formula, R₁, R₃, R₄, R₅ may be independently selected from C₁₂-C₂₂ hydrocarbyl or C₁₂-C₂₂ substituted hydrocarbyl groups. The C₁₂-C₂₂ hydrocarbyl or C₁₂-C₂₂ substituted hydrocarbyl groups may be saturated or unsaturated, linear or branched, cyclic or acyclic. In one embodiment R₁, R₃, R₄, R₅ may be identical. In another embodiment of the present invention, R₁ and R₃, or R₁, R₄ and R₅ may be independently selected from the group consisting of C₁₂-C₂₂ alkyl, alkenyl, hydroxyalkyl or alkoxyl group.

By "solid" as used herein, it is meant that the soil adhering compound(s) useful in the present invention has an average melting point of at least about 30°C or at least about 35°C or at least about 40°C. With such a melting point, the soil adhering compound(s) is relatively immobile and remains localized on the substrate at room temperature (e.g., 20-25°C). Suitably the soil adhering compound(s) is non-migratory once applied to the substrate. The soil adhering compound(s) is substantially not transferable to the user.

The substrate useful in the present invention may comprise one soil adhering compound of the above formula or it may comprise a mixture of soil adhering compounds of the above formula. The soil adhering compound(s) may also be mixed with other ingredients to form a composition as disclosed herein below.

In a preferred embodiment, the substrate useful in the present invention comprises at least a soil adhering compound of the formula:

R₁-CO-[A]-R₂

wherein R₁ is a C₁₂-C₂₂ hydrocarbyl or C₁₂-C₂₂ substituted hydrocarbyl group,
wherein R₂ is selected from:
   (a) -O-CO-R₃ wherein R₃ is a C₁₂-C₂₂ hydrocarbyl or C₁₂-C₂₂ substituted hydrocarbyl group; or
   (b) 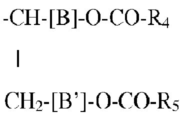
      wherein R₄ is a C₁₂-C₂₂ hydrocarbyl or C₁₂-C₂₂ substituted hydrocarbyl group;
      wherein R₅ is a C₁₂-C₂₂ hydrocarbyl or C₁₂-C₂₂ substituted hydrocarbyl group;
wherein A, B and B' are independently selected from a polymer or copolymer of ethylene oxide, propylene oxide, butylene oxide wherein said polymer or said copolymer comprises from 2 to 200 monomers.

In another preferred embodiment, the substrate comprised in the personal care product of the present invention comprises at least a soil adhering compound of the formula:

R₁-CO-[A]-O-CO-R₃

wherein R₁ is a C₁₂-C₂₂ hydrocarbyl or C₁₂-C₂₂ substituted hydrocarbyl group,
wherein R₃ is a C₁₂-C₂₂ hydrocarbyl or C₁₂-C₂₂ substituted hydrocarbyl group,
wherein A is a polymer or copolymer of ethylene oxide, propylene oxide, butylene oxide wherein said polymer or said copolymer comprises from 2 to 200 monomers.

In the above formula, R₁ and R₃ may be identical or may be different. R₁ and R₃ may be independently selected from C₁₂-C₂₂ hydrocarbyl or C₁₂-C₂₂ substituted hydrocarbyl groups. The C₁₂-C₂₂ hydrocarbyl or C₁₂-C₂₂ substituted hydrocarbyl groups may be saturated or unsaturated, linear or branched, cyclic or acyclic. In preferred embodiments, R₁ and R₃ may be independently selected from the group consisting of C₁₂-C₂₂ alkyl, C₁₂-C₂₂ alkenyl, C₁₂-C₂₂ hydroxyalkyl or C₁₂-C₂₂ alkoxyl group.

Preferred soil adhering compounds include alkoxylated fatty diester such as ethoxylated fatty diester or propoxylated fatty diester (e.g., diester of polyethylene glycol, or diester of polypropylene glycol). Hereby, it may be preferred that the number of monomers (c.g, ethylene oxide or propylene oxide) is comprised between 2 and 200, or between 70 and 190, or between 100 and 180. Preferred alkoxylated fatty diesters include alkoxylated distearate ester, alkoxylated diisostearate ester or alkoxylated dibehenate ester.

Suitable examples of soil adhering compound include ethoxylated fatty diesters such as PEG-150 distearate (R₁=R₃=C₁₇H₃₅) (available from Global Seven, Franklin, New Jersey, USA), PEG-175 diisostearate (R₁=R₃=C₁₇H₃₅) (available from Global Seven, Franklin, New Jersey, USA), PEG-150 dibehenate (available from Global Seven, Franklin, New Jersey, USA), PEG-90 distearate (available from Lubrizol, Cleveland, Ohio, USA), PEG-2 distearate (available from A&E Connock, Hampshire, England), PEG-4 distearate (available from Protameen, Totowa, New Jersey, USA) and PEG-20 distearate (available from A&E Connock, Hampshire, England). Preferred soil adhering compounds include alkoxylated fatty triester such as ethoxylated fatty triester or propoxylated fatty triester (e.g., triester of polyethylene glycol, or triester of polypropylene glycol). Hereby, it may be preferred that the number of monomers (e.g, ethylene oxide or propylene oxide) is comprised between 2 and 200, or between 70 and 190, or between 100 and 180. Suitable example includes PEG-150 polyglyceryl-2 tristearate.

The soil adhering compound(s) of the formula as disclosed above may be used for imparting adherence for body exudates to a substrate. In this regard, a method for imparting adherence for body exudates to a substrate comprised by a personal care product as defined in claim 9 is provided. The method comprises the step of applying to a substrate at least a solid nonionic compound of the formula:

R₁-CO-[A]-R₂

wherein R₁ is a C₁₂-C₂₂ hydrocarbyl or C₁₂-C₂₂ substituted hydrocarbyl group;
wherein R₂ is selected from:
   (a) -O-CO-R₃ wherein R₃ is a C₁₂-C₂₂ hydrocarbyl or C₁₂-C₂₂ substituted hydrocarbyl group; or
   (b) 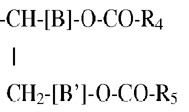
      wherein R₄ is a C₁₂-C₂₂ hydrocarbyl or C₁₂-C₂₂ substituted hydrocarbyl group;
      wherein R₅ is a C₁₂-C₂₂ hydrocarbyl or C₁₂-C₂₂ substituted hydrocarbyl group;
wherein A, B and B' are independently selected from: a polymer or copolymer of ethylene oxide, propylene oxide, butylene oxide, hydroxypropylene oxide wherein said polymer or said copolymer comprises from 2 to 200 monomers.

### OPTIONAL INGREDIENTS

The soil adhering compound or mixture of soil adhering compounds useful in the present invention may be used alone or may be combined with optional ingredients to form a composition.

The composition suitable in the present invention may comprise ingredients such as, but not limited to, emollients, immobilizing agent, surfactants and/or emulsifiers, buffering agents and mixture thereof. The composition suitable in the present invention may also include optional ingredients providing additional skin care benefits.

Compositions useful in the present invention are preferably hydrophilic.

In one embodiment, the compositions may be non-migratory once applied to the substrate.

They are preferably substantially non-transferable to the user.

The compositions useful in the present invention (typically applied to or on, or impregnated in or on a substrate) comprises at least a soil adhering compound in an amount ranging from 10 % to 99 %, or from 30% to 97%, or from 50% to 95%, or from 60 to 90% by weight of the composition.

The optional ingredients and their amount included in the composition will depend on a variety of factors, including the end use of the composition (e.g. composition applied to or on the topsheet of a diaper, of a sanitary napkin.

### Emollient

In some embodiments, the compositions useful in the present invention may comprise from 5% to 90% of one or more emollients by weight of the composition. An emollient, as used herein, is a material that softens, soothes, supples, coats, lubricates, moisturizes or cleanses the skin. In one embodiment, the composition does not comprise any emollient.

Representative emollients useful in the present invention include, but arc not limited to, emollients that are petroleum-based, and/or liquid polyethylene glycol having a molecular weight (weight average) which is below 800, or below 600, or below 400 and derivatives thereof, those derivatives being end-capped esters and ethers derivatives such as methyl, ethyl and/or propyl end-capping group(s). Suitable petroleum-based emollients include hydrocarbons, having chain length of from 16 to 32 carbon atoms. Petroleum-based hydrocarbons having these chain lengths include mineral oil (also known as "liquid petrolatum") and petrolatum (also known as "mineral wax", "petroleum jelly" and "mineral jelly"). Suitable polyethylene glycols include PEG-8.

### Immobilizing agent

In some embodiments, the compositions useful in the present invention may comprise from 5% to 50%, most preferably from 10 to 40% of one or more immobilizing agents by weight of the composition. An immobilizing agent, as used herein, is a material that helps in stabilizing the composition on the substrate and limiting its transfer to the skin.

Immobilizing agents useful in the present invention include waxes such as carnauba, ozokerite, beeswax, candelilla, paraffin, ceresin, esparto, rezowax, isoparaffin and other known mined and mineral waxes.

Other suitable immobilizing agents for the present invention can comprise a member selected from the group consisting of C₁₄-C₂₂ fatty alcohols, C₁₂-C₂₂ fatty acids, and C₁₄-C₂₂ ethoxylated fatty alcohol and C₁₂-C₂₂ ethoxylated fatty acids having an average degree of ethoxylation ranging from 2 to about 30, and mixtures thereof. Preferred immobilizing agents include C₁₆-C₁₈ fatty alcohols, most preferably selected from the group consisting of cetyl alcohol, stearyl alcohol, and mixtures thereof. Mixtures of cetyl alcohol and stearyl alcohol are particularly preferred. Other preferred immobilizing agents include C₁₆-C₁₈ fatty acids, most preferably selected from the group consisting of palmitic acid, stearic acid, and mixtures thereof. Mixtures of palmitic acid and stearic acid are particularly preferred. Still other preferred immobilizing agents include C₁₆-C₁₈ fatty alcohol ethoxylates having an average degree of ethoxylation ranging from about 5 to about 20. Preferably, the fatty alcohols, fatty acids and fatty alcohols are linear.

### Additional surfactants

In one embodiment, the compositions useful in the present invention, may comprise from 1% to 75% or from 2% to 60% or from 10% to 50% of one or more additional surfactants by weight of the composition. However, in one embodiment, the compositions do not comprise any additional surfactant, in particular when used in absorbent articles.

The surfactant or combinations of surfactants may be mild, which means that the surfactants provide sufficient cleansing or detersive benefits but do not overly dry or otherwise harm or damage the skin.

A wide variety of surfactants are useful herein and include those selected from the group consisting of anionic surfactants, nonionic surfactants, cationic surfactants, amphoteric surfactants, zwitterionic surfactants, and mixtures thereof.

Non-limiting examples of anionic surfactants include those selected from the group consisting of sarcosinates, sulfates, sulfonates, isethionates, taurates, phosphates, lactylates, glutamates, and mixtures thereof. Amongst the isethionates, the alkoyl isethionates are useful, and amongst the sulfates, the alkyl and alkyl ether sulfates are useful. Other anionic materials useful herein are soaps (i.e., alkali metal or amine salts, e.g., sodium, potassium or triethanolamine salts) of fatty acids, typically having from about 8 to about 24 carbon atoms.

Nonionic surfactants useful herein include those selected from the group consisting of alkyl glucosides, alkyl polyglucosides, polyhydroxy fatty acid amides, amine oxides, and mixtures thereof.

Suitable amphoteric or zwitterionic surfactants for use in the compositions herein include those which are known for use in hair care or other personal care cleansing. Amphoteric surfactants suitable for use in the present compositions are well known in the art and include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group such as carboxy, sulfonate, sulfate, phosphate, or phosphonate. Useful amphoteric surfactants include, but are not limited to, the group consisting of cocoamphoacetate, cocoamphodiacetate, lauroamphoacetate, lauroamphodiacetate, and mixtures thereof. Zwitterionic surfactants suitable for use herein include those surfactants broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. Useful zwitterionic detersive surfactants are the betaines, amphoacetates and sulfobetaines, e.g., cocoamidopropylbetaine, sodium laurylamphoacetate and cocoamidopropylhydroxysultaine.

### Buffering agent

In one embodiment, the compositions useful in the present invention may comprise from 0.1% to 5% or from 0.5 to 3% or from 1 to 2% of one or more buffering agents by weight of the composition.

Suitable buffering agents for use in the present composition include, but are not limited to, calcium carbonate, sodium citrate, urea, glycine, triacetin, monoalkylphosphates, monoalkyl citrates, disodium phosphate, fruit acids (e.g., malic acid), citric acid, citrate and any combinations thereof. Suitably, the pH of the composition may be from about pH 3, 4, or 5 to about pH 7, 7.5, or 8.

### Other optional ingredients

The compositions useful in the present invention may comprise other optional ingredients. These optional ingredients include solvents, diluents, viscosity modifiers, perfumes, antibacterial actives, pharmaceutical actives, film formers, deodorants, opacifiers, pigments, dyes, and coloring agents, astringents, solvents and the like. All these materials are well known in the art as additives for such compositions and can be employed in appropriate amounts in the compositions of the present invention.

### APPLYING THE SOIL ADHERING COMPOUND(S) OR A COMPOSITION COMPRISING THE SOIL ADHERING COMPOUND(S) TO THE SUBSTRATE

The substrates comprised by the personal care product of the present invention comprise at least a soil adhering compound imparting adherence for body exudates such as feces and menses to said substrate. By "substrates comprise" or "substrate comprising" herein, it is meant that the substrate is treated by said soil adhering compound(s) or said composition comprising the soil adhering compound(s). By "treated" it is meant that the soil adhering compound(s) of the present invention are applied to the substrate. The soil adhering compound(s) or the composition comprising the soil adhering compound(s) can be applied to the substrate by any known or otherwise effective technique for distributing a compound or composition onto a substrate.

### Substrate comprised by, or forming, or used for manufacturing of, an absorbent article

Non-limiting examples of methods of applying the soil adhering compound(s) or a composition comprising the soil adhering compound(s) to a substrate for use in an absorbent article include spraying, printing (e.g., flexographic printing), coating (e.g., contact slot coating and gravure coating), extrusion, or combinations of these application techniques.

The minimum amount of soil adhering compound(s) or of a composition comprising the soil adhering compound(s) applied to the substrate is an amount effective for imparting adherence for body exudates such as feces and menses to the substrate. The soil adhering compound(s) or the composition comprising the soil adhering compound(s) is applied to the substrate in an amount ranging from about 0.05 mg/cm² to 6.4 mg/cm², preferably from about 0.1 mg/cm² to 3 mg/cm² or from about 0.8 mg/cm² to 2.5 mg/cm² (mg of composition per square centimeter of coated substrate).

The soil adhering compound(s) or a composition comprising the soil adhering compound(s) useful in the present invention may be applied to a substrate comprised by, or forming or used for manufacturing of the topsheet and/or the leg cuffs and/or the barrier cuffs and/or the wings and/or the side flaps as liquid or as a semi-liquid. Typically, the soil adhering compound(s) or a composition comprising the soil adhering compound(s) is applied to the outer surface or outer surfaces that in use are in contact with the skin of the wearer, i.e. the soil adhering compound(s) or a composition comprising the soil adhering compound(s) is applied to the body facing surface. The soil adhering compound(s) or the composition comprising the soil adhering compound(s) is typically applied as a melt thereof to the substrate comprised by, or forming, or used for manufacturing of the topsheet and/or the leg cuffs and/or the barrier cuffs and/or the wings and/or the side flaps of an absorbent article. Since the soil adhering compound(s) or the composition comprising the soil adhering compound(s) preferably melts at above-ambient temperatures, it is usually heated and applied to the substrate part of, or constituting the topsheet and/or the leg cuffs and/or the barrier cuffs and/or the wings and/or the side flaps as a liquid or semi-liquid. Typically, the soil adhering compound(s) or the composition comprising the soil adhering compound(s) is heated to a temperature in the range from about 40° to about 100°C, preferably from 50° or from 60°C or even from to 90°C to about 100°C, prior to being applied to the substrate part of or constituting the topsheet and/or the leg cuffs and/or the barrier cuffs and/or the wings and/or the side flaps. Then, once the soil adhering compound(s) or the composition comprising the soil adhering compound(s) has been applied to the substrate part of, or constituting the topsheet and/or the leg cuffs and/or the barrier cuffs and/or the wings and/or the side flaps, it is allowed to cool and solidify; it may thereby form a coating on the surface of the substrate.

The soil adhering compound(s) or the composition comprising the soil adhering compound(s) can be applied uniformly or non-uniformly to the body facing surface of substrate. By nonuniform it is meant here the amount, pattern of distribution of the soil adhering compound(s) or the composition comprising the soil adhering compound(s) can vary over the substrate; including for example the embodiment whereby one or more portions of the treated surface of the substrate herein can have a greater amount of the soil adhering compound(s) or of the composition comprising the soil adhering compound(s) than other one or more other portions (e.g. some portions comprise a higher basis weight of the soil adhering compound(s) or of the composition comprising the soil adhering compound(s) than other portions, such as for example at least 10% or at least 20% higher), including portions of the surface that do not have any soil adhering compound or any composition comprising the soil adhering compound(s) on them. Such portions may have any of the dimensions as for example defined below. As the soil adhering compound(s) or the composition comprising the soil adhering compound(s) useful in the present invention imparts adherence for body exudates such as feces or menses, it may be, for example, comprised in those portions of the substrate comprised by, or forming, or used for manufacturing of the topsheet or the cuffs, which lie adjacent the skin areas of the wearer, which typically are contaminated with feces and menses. Thus, the soil adhering compound(s) or the composition comprising the soil adhering compound(s) could for example be comprised in those portions which lie adjacent the buttocks and/or the whole groove length of the wearer in use, preferably also in the region of the genitals. The amount of soil adhering compound(s) or the composition comprising the soil adhering compound(s) applied can be the same for the rear third of the article (i.e. a third of the longitudinal extension of the absorbent articles starting from the outer edge of the chassis in the rear waist region), the central third of the article and the front third of the article. Alternatively, the amount of soil adhering compound(s) or the composition comprising the soil adhering compound(s) applied can be different for the rear, central and front third of the absorbent article.

Where the soil adhering compound(s) or the composition comprising the soil adhering compound(s) is applied non-uniformly, it can be applied intermittently, i.e. discontinuously. Any pattern, including pattern of portions, as described herein above, comprising the soil adhering compound(s), may be utilized, including, for example, application of a pattern of (or a pattern of portion having the shape of) small droplets (obtained via, e.g., spraying), discrete figures of any shape or size, such as round, oval, rectangular, triangular, star-shaped, heart-shaped or shaped in the form of an animal (obtained via, e.g., gravure printing), alternating stripes that run in the longitudinal or lateral direction of the article, etc. Also, the substrate can comprise figures of different shapes and/or of different sizes. By alternating stripes, it is meant portions in which the soil adhering compound(s) or the composition comprising the soil adhering compound(s) is applied as stripes separated by portions which have no soil adhering compound or no composition comprising the soil adhering compound(s) applied.

The portions, stripes and/or other discrete figures and/or droplets may have a width from between 0.1mm to about 50mm, from between 0.1 to about 30mm, from between 0.5mm to about 50mm, from about 0.5mm to about 40mm, from between 2mm to about 40mm, from between 2mm to about 20mm, from between 2mm to about 15mm, or from between 5mm to about 20mm. The spacing between the stripes having no soil adhering compound or no composition comprising the soil adhering compound(s) applied may have a width from between 0.1mm to about 100mm, from about 0.1mm to about 50mm, from between 0.1 to about 30mm, from between 0.5mm to about 50mm, from about 0.5mm to about 40mm, from between 2mm to about 40mm, from between 2mm to about 20mm, from between 2mm to about 15mm, or from between 5mm to about 20mm. The portions in the form of stripes and/or discrete figures and/or droplets may have a length of at least 0.5mm, or of at least 2mm or of at least 5mm. In one embodiment, if the portions comprising the soil adhering compound(s) or the composition comprising the soil adhering compound(s) are in the form of stripes, they may extend from one edge of the substrate to the opposite edge. Preferably, the stripes may extend into the rear waist region of the substrate to the extent that they also cover the buttocks and/or most of the groove length. Also, the number or the density of the stripes may be higher in those portions lying against the areas typically affected with feces smeared against the skin. Further, the basis weight of the stripes may be higher, such as 10% higher or 20% higher in those portions lying against the areas typically affected with feces smeared against the skin.

The soil adhering compound(s) or the composition comprising the soil adhering compound(s) can be applied directly to the substrate comprised by, or forming, or used for manufacturing of, the topsheet and/or leg cuff and/or barrier cuff and/or the wings and/or the side flaps of an absorbent article or it may be applied to another material or component which is then adhered to the desired portion of the absorbent article (such as a calender roll).

If the soil adhering compound(s) or the composition comprising the soil adhering compound(s) is applied in the form of figures, the density (i.e. dots lying closer together) and/or the size of the figures and/or the basis weight of the soil adhering compound(s) or the composition comprising the soil adhering compound(s) comprised by the figures may be higher, such as 10% higher or 20% higher in those portions lying against the areas (e.g., the central third and rear third of the absorbent article) typically affected with feces smeared against the skin.

In one embodiment, the soil adhering compound(s) or the composition comprising the soil adhering compounds may also be applied to the acquisition layer or to the absorbent core of an absorbent article.

### PERSONAL CARE PRODUCTS COMPRISING THE TREATED SUBSTRATE

The substrates comprising at least a soil adhering compound are used to manufacture personal care products. Personal care products comprising the substrates useful in the present invention are absorbent articles (incontinence products such as infant or adult diapers or adult incontinence products, feminine hygiene products).

### Incontinence product

In the following, a diaper is described as one embodiment of an incontinence product. However, as the skilled person is aware of, most of the components and materials described herein below are also applicable to other incontinence products such as pant-like diapers or adult incontinence products.

The diaper has a longitudinal axis and a transverse axis. The diaper has further an inner, body facing surface and an outer, garment facing surface opposed to the inner surface.

One end portion of the diaper is configured as a front waist region (which is the front one third of the article, having one third of the length of the article). The opposite end portion is configured as a back waist region (back one third) of the diaper, having one third of the length of the article. An intermediate portion of the diaper is configured as a crotch region (centre one third), which extends longitudinally between the front and back waist regions, also having one third of the length of the article. The crotch region is that portion of the diaper which, when the diaper is worn, is generally positioned between the wearer's legs.

The chassis of the diaper comprises the main body of the diaper. The chassis comprises typically a topsheet, which may be liquid pervious, and which may, for the purpose of the invention, comprise or be made of said substrate comprising at least a soil adhering compound as described herein. The chassis typically also comprises a backsheet. The chassis further includes an absorbent core encased between the topsheet and the backsheet. Said backsheet may typically be a liquid impervious backsheet, as known in the art. In one embodiment, the liquid impervious backsheet comprises a thin plastic film such as a thermoplastic film having a thickness of about 0.01 mm to about 0.05 mm. Suitable backsheet materials comprise typically breathable material, which permit vapors to escape from the absorbent article while still preventing exudates from passing through the backsheet. Suitable backsheet films include those manufactured by Tredegar Industries Inc. of Terre Haute, IN and sold under the trade names X15306, X10962 and X10964. The backsheet, or any portion thereof, may be elastically extendable in one or more directions. The absorbent core mentioned above may comprise any absorbent material that is generally compressible, conformable, non-irritating to the wearer's skin, and capable of absorbing and retaining liquids such as urine and other body exudates.

The absorbent core may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding, meltblown polymers including coform, cross-linked cellulosic fibers, tissue including tissue wraps and tissue laminates, absorbent foams, absorbent sponges, superabsorbent polymers, absorbent gelling materials, or any equivalent material or combinations of materials. The configuration and construction of the absorbent core may also be varied, e.g. the absorbent core may have varying caliper zones, a hydrophilic gradient, a superabsorbent gradient, or lower average density and lower average basis weight acquisition zones; or may comprise one or more layers or structures. The total absorbent capacity of the absorbent core should, however, be compatible with the design loading and the intended use of the diaper. Further, the size and absorbent capacity of the absorbent core may be varied to accommodate wearers ranging from infants through adults.

The diapers, pant-like diapers or adult incontinence diaper or pad herein typically have also leg cuffs and/ or barrier cuffs, which may, for the purpose of the invention, comprise or be made of said substrate of the invention, as described herein. Typically, it may have a pair of opposing (elasticated) leg cuffs, including so-called side panels, and/ or a pair of opposing (elasticated) barrier cuffs that provide improved containment of liquids and other body exudates. The cuffs of a pair may be mirror images of one another in the y-axis (longitudinal axis) of the article. Suitable cuffs are described in for example U.S. 3,860,003; U.S 4,808,178 and 4,909; U.S. 4,695,278 and 4,795,454. The cuffs may also be made of nonwoven materials as described above and they are preferably hydrophobic.

The backsheet may be directly or indirectly attached to or joined with the topsheet herein and/ or the barrier and/ or leg cuffs herein.

Further, the diaper may comprise a front and back waist band and/ or a fastening system, typically joined to the waistband, as known in the art. Preferred fastening systems comprise fastening tabs and landing zones, wherein the fastening tabs are attached or joined to the back region of the diaper and the landing zones are part of the front region of the diaper.

### Feminine hygiene product

In the following, a feminine hygiene product is described (e.g., sanitary napkin or panty-liner). A feminine hygiene product may comprise a topsheet which may, for the purpose of the invention, comprise or be made of said substrate comprising at least a soil adhering compound as described herein, a backsheet, and an absorbent core positioned between the topsheet and backsheet; each component having a body facing surface and a garment facing surface. The topsheet may be made of a substrate comprising at least a soil adhering compound as described herein. The backsheet can be any known or otherwise effective backsheet material, provided that the backsheet prevents external leakage of exudates absorbed and contained in the feminine hygiene article. Flexible materials suitable for use as the backsheet include, but are not limited to, woven and nonwoven materials, laminated tissue, polymeric films such as thermoplastic films of polyethylene and/or polypropylene, composite materials such as a film-coated nonwoven material, or combinations thereof, as is well known in the art of making feminine hygiene articles such as sanitary napkins, pantiliners, and the like.

The feminine hygiene product also comprises an absorbent core. The absorbent core is typically positioned between the topsheet and the backsheet. The size and shape of the absorbent core can be altered to meet absorbent capacity requirements, and to provide comfort to the wearer/user. The absorbent core suitable for use in the present invention can be any liquid-absorbent material known in the art for use in absorbent articles, provided that the liquid-absorbent material can be configured or constructed to meet absorbent capacity requirements. Non-limiting examples of liquid-absorbent materials suitable for use as the absorbent core include comminuted wood pulp which is generally referred to as airfelt; creped cellulose wadding; absorbent gelling materials including superabsorbent polymers such as hydrogel-forming polymeric gelling agents; chemically stiffened, modified, or cross-linked cellulose fibers; meltblown polymers including coform; synthetic fibers including crimped polyester fibers; tissue including tissue wraps and tissue laminates; capillary channel fibers; absorbent foams; absorbent sponges; synthetic staple fibers; peat moss; or any equivalent material; or combinations thereof, as is well known in the art of making feminine hygiene articles such as sanitary napkins, pantiliners, and the like.

The feminine hygiene product may also comprise as cuffs so-called wings which may enable attachment to the underwear of the wearer. The cuffs may be made of or comprise a substrate comprising at least a soil adhering compound. The sanitary napkins and/ or panty-liners herein may preferably comprise a fastening means comprised by the backsheet and/ or by the wings (cuffs). Preferred are adhesive attachment means that are present on or attached to at least the backsheet.

### COMBINATION OF ABSORBENT ARTICLES AND WIPES

### Method for improving the cleaning of the skin

Cleaning the skin in the area covered by absorbent articles can be improved by the combined use of an absorbent article such as those described herein (e.g., diaper including a topsheet comprising a substrate comprising at least a soil adhering compound) with a wipe comprising a lotion that reduces the adherence of body exudates to the user's body.

In this regard, a method for improving the cleaning of the skin in the area that can be contacted with feces or menses (such as the genitals or the buttocks) is provided. The method comprises the steps of:
(a) Delivering to the skin that may be contacted with feces or menses a lotion reducing or preventing the adhesion of feces or menses to the skin, said lotion being comprised by a wipe,
(b) Applying to said skin an absorbent article comprising a substrate comprising at least a soil adhering compound.

Such a combined use improves the cleaning capabilities. Indeed, the wipe delivers a lotion that reduces or prevents the adherence of the feces or menses to the skin whereas the absorbent article applied subsequently on the skin exhibits adherence for feces or menses, thus helping to keep the skin free of exudates.

In one embodiment, the soil adhering compound(s) or preferably a composition comprising the soil adhering(s) in the form of a cream, paste, lotion or mousse may be applied directly to the surface of the wipe by the caregiver at the point of use.

Alternatively, a method (not within the scope of the present invention) for improving the cleaning of the skin in the area that can be contacted with feces or menses (such as the genitals or the buttocks) may comprise the steps of:
(a) Delivering to the skin that may be contacted with feces or menses a lotion reducing or preventing the adhesion of feces or menses to the skin, said lotion being comprised by an absorbent article,
(b) Applying to said skin a wipe comprising a substrate comprising at least a soil adhering compound.

The lotion reducing or preventing the adhesion of feces or menses to the skin comprised by the wipe of the present method may be an aqueous or a non-aqueous lotion. Suitable lotions include anti-stick agents such as, but not limited to:
- Non-polymeric anti-stick agents such as glycerol and related polyols such as sorbitol, maltitol, xylitol, pentaerythitol, sucrose, glucose, maltose, maltotriose, maltodextrin, maltopentose, maltohexose, and isomaltulose, ethylene glycol, propylene glycol, butylene glycol, and the like,
- Polymeric anti-stick agents comprising polyethylene glycol, polypropylene glycol, polybutylene glycol, polyglycerol or mixtures thereof, including block copolymers comprising ethylene oxide and propylene oxide, and the like,
- Alkoxylated polyol compounds,
- Phosphate compounds such as mono-alkyl phosphates and di-alkyl -phosphates,
- Silicone copolyols comprising polyethylene glycol, polypropylene glycol, polybutylene glycol, polyglycerol or mixtures thereof,
- mono-functional heteroatom oxide ethers of polyethylene glycol, polypropylene glycol, polybutylene glycol, polyglycerol or mixtures thereof, and homologues thereof,
- poly-functional heteroatom oxide esters of polyethylene glycol, polypropylene glycol, polybutylene glycol, polyglycerol or mixtures thereof, and homologues and derivatives thereof, or any combinations thereof.

Other suitable anti-stick agents are disclosed in US 2007/0286894 A1 and US 20007/0286893A1.

The lotion reducing or preventing the adhesion of feces or menses to the skin comprised by the absorbent article of the present method, may comprise at least a liquid compound and a solid compound as follows: it may comprise a liquid compound (at 20-25°C) selected from the group consisting of: propyl ether, dipropylene glycol butyl ether, methyl propanediol, ethylene glycols, diethylene glycols, propylene glycols, dipropylene glycols, glycerin, sorbitol, hydrogenated starch hydrolysate, polyethylne glycol or derivatives, polyethylene glycol derived surfactants, polypropylene derived surfactants, ethylene glycol or derivatives, diethylene glycol or derivatives and dipropylene glycol or derivatives; or mixtures thereof. Preferred may also be liquid polyethylene glycols (PEG's) and liquid Polypropylene glycols (PPG) and derivatives thereof, said PEG's and PPG's having a molecular weight (weight average) which is from 100 to less than 720, preferably from 100 or 350 to 700. Typical liquid polyethylene glycols are known as PEG-4, PEG-6, PEG-7, PEG-8, PEG-9, PEG-10, PEG-12 and PEG-14. Preferred liquid derivatives are mono and/ or di-end capped PEG's and PPg's, such as for example a polyethylene glycol monomethyl ether, such as available as Polyglykol M400 from Clariant Corporation. A preferred liquid PEG includes also a Polyethylene glycol dimethyl ether with a MW of 500 (available from Sigma Aldrich).

Also useful are liquid ethylene oxide - propylene oxide copolymers and polyethylene - polypropylene block copolymers (EO-PO block copolymers), such as Genapol PF10- a EO-PO block copolymer from Clariant Corp.)

In addition to said liquid compound, the lotion composition comprises a second, solid compound (at 20-25°C) of the group including: solid polyethylene glycol and end-capped derivatives thereof; solid polypropylene glycol and end-capped derivatives thereof; solid nonionic surfactants with HLB value of at least 10; solid fatty compounds selected from the group consisting of solid fatty acids, solid fatty soaps and solid fatty alcohols; ethoxylated natural fats or propoxylated natural fats, such as PEG-150 jojoba.

Exemplary further lotion compositions may be such that:
said first liquid compound may be a liquid polyethylene glycol and said second compound may be a solid nonionic alkoxylated (e.g. ethoxylated) fatty alcohol, then the HLB value is at least 13; or
said first compound may be may a liquid fatty acid ester comprising at least one fatty acid unit and at least one ethylene glycol unit and said second compound may be a solid polyethylene glycol; or
said first compound may be a liquid polyethylene glycol and said second compound is a solid fatty compounds selected from the group consisting of solid fatty acids and solid fatty soaps and solid fatty alcohols.

In one embodiment the solid compound may a solid nonionic surfactant, preferably a solid polyethylene glycol fatty alcohol ethers having the general formula R(OCH2CH2)ₙOH, where R represents an alkyl group or a blend of alkyl groups, with for example 8 to 30 or 12 to 22 carbon atoms, and n is the degree of ethoxylation, e.g. 2 to 200. It may be preferred, that the lotion composition comprises from 20% to 80% by weight, or 30% to 70% by weight, or 40% to 60% by weight of this liquid polyethylene glycol, for example 50% by weight of Steareth-100.

### Combination absorbent articles and wipes

It can be provided a combination of a diaper including a topsheet comprising a substrate comprising a soil adhering compound with wipes comprising at least a soil adhering compound or with wipes comprising an anti-stick agent.

This combination includes combined uses or sales. The absorbent articles comprising a substrate comprising at least a soil adhering compound may be packaged with one or more wipes. In one embodiment, one or more wipes may be packaged in a first package and one or more absorbent articles may be packaged in a second package. The first and second packages may be packaged together or they may be held in assembly by any means. In another embodiment, one absorbent article may be packaged with one or more wipes as one individual package, which is especially convenient for users en-route, where it might be desirable to carry only one absorbent article and one or more wipes. The absorbent articles and the wipes may also be co-marketed, e.g, designed and/or advertised to be sold and/or used together.

A suitable combination includes the combination of absorbent article(s) comprising a substrate comprising the soil adhering compound(s) with at least one wipe including or made of a substrate comprising the soil adhering compound(s).

A suitable combination includes the combination of absorbent article(s) comprising a substrate comprising the soil adhering compound(s) with at least one wipe comprising a lotion that prevents or reduces the adherence of the feces or menses to the skin. The lotion preventing or reducing the adherence of the feces or menses to the skin may include one of the anti-stick agents as disclosed herein above.

### SUBSTRATE AND PERSONAL CARE PRODUCT COMPRISING A FURTHER COMPOSTION

The substrate comprised by the personal care product of the present invention may have one or more further compositions disposed thereon. By "further composition" as used herein, it is meant a composition that does not comprise the soil adhering compound. The compositions may have different formulations such that the substrate can be designed to deliver specific benefit to specific portions of the skin of the user.

In one embodiment, the substrate, where comprised by, or forming, or used for manufacturing of, a topsheet may comprise one or more portions, as defined above, or regions comprising at least a soil adhering compound and one or more portions comprising a composition reducing or preventing the adhesion of exudates to the skin such as the ones disclosed herein above. For instance, the portion of the substrate in contact with the genitals such as the front third of the absorbent article may comprise the soil adhering compound(s) or the composition comprising the soil adhering compound(s) whereas the portion of the substrate in contact with the buttocks such as the central and rear third of the absorbent article may comprise a composition reducing or preventing the adhesion of exudates to the skin. Alternatively, the portion of the substrate in contact with the genitals such as the front third of the absorbent article may comprise the composition reducing or preventing the adhesion of exudates to the skin whereas the portion of the substrate in contact with the buttocks such as the central and rear third of the absorbent article may comprise the soil adhering compound(s).

The amounts, patterns of distribution of the soil adhering compound(s) and of the further composition may vary depending on the substrate. Any of the methods disclosed herein above herein apply.

### SPECIFIC ILLUSTRATIONS OF THE PRESENT INVENTION

### Example 1

### A. Preparation of the composition

The composition is made by mixing the following melted (i.e., liquid) components together:
50g PEG-150 distearate
50g PEG-8

### B. Preparation of the treated diaper

The composition of example 1 is placed into a heated tank operating at a temperature of 160°F. The composition is subsequently sprayed (using a Dynatec E84B1758 spray head, operating at a temperature of 165°F and an atomization pressure of 2.40 psig) onto the topsheet of a diaper in a 3.75 inch wide (diaper lateral direction) and 7 inch long (diaper longitudinal direction) area, the patch beginning 1 inch forward of the lateral centerline and extending toward the rear of the product. Add-on level = 0.93 mg/cm².

### Example 2

### A. Preparation of the composition

The composition is made by mixing the following melted (i.e., liquid) components together:
50g PEG-175 diisostearate
50g PEG-8

### B. Preparation of the treated diaper

The composition of example 2 is placed into a heated tank operating at a temperature of 160°F. The composition is subsequently sprayed (using a Dynatec E84B1758 spray head, operating at a temperature of 165°F and an atomization pressure of 2.40 psig) onto the topsheet of a diaper in a 3.75 inch wide (diaper lateral direction) and 7 inch long (diaper longitudinal direction) area, the patch beginning 1 inch forward of the lateral centerline and extending toward the rear of the product. Add-on level = 0.93 mg/cm².

### Example 3

### A. Preparation of the composition

The composition is made by mixing the following melted (i.e., liquid) components together:
50g PEG-150 polyglyceryl-2 tristearate
50g PEG-8

### B.Preparation of the treated diaper

The composition of example 3 is placed into a heated tank operating at a temperature of 160°F. The composition is subsequently sprayed (using a Dynatec E84B1758 spray head, operating at a temperature of 165°F and an atomization pressure of 2.40 psig) onto the topsheet of a diaper in a 3.75 inch wide (diaper lateral direction) and 7 inch long (diaper longitudinal direction) area, the patch beginning 1 inch forward of the lateral centerline and extending toward the rear of the product. Add-on level = 0.93 mg/cm².

## Claims

1. A personal care product wherein the personal care product is selected from a diaper, a feminine hygiene product and an adult incontinence product, the personal care product comprising a substrate comprising at least a soil adhering compound imparting adherence for exudates to said substrate, or a composition comprising at least the soil adhering compound in an amount ranging from 10% to 99%, said compound being a solid non-ionic compound of formula:
R₁-CO-[A]-R₂
wherein R₁ is a C₁₂-C₂₂ hydrocarbyl or C₁₂-C₂₂ substituted hydrocarbyl group;
wherein R₂ is selected from:
(a) -O-CO-R₃ wherein R₃ is a C₁₂-C₂₂ hydrocarbyl or C₁₂-C₂₂ substituted hydrocarbyl group; or
(b) 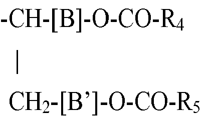
wherein R₄ is a C₁₂-C₂₂ hydrocarbyl or C₁₂-C₂₂ substituted hydrocarbyl group;
wherein R₅ is a C₁₂-C₂₂ hydrocarbyl or C₁₂-C₂₂ substituted hydrocarbyl group;
wherein A, B and B' are independently selected from:
a polymer or copolymer of ethylene oxide, propylene oxide, butylene oxide, hydroxypropylene oxide wherein said polymer or said copolymer comprises from 2 to 200 monomers,
wherein the soil adhering compound or the composition comprising the soil adhering compound is applied to the substrate in an amount ranging from 0.05 mg/cm² to 6.4 mg/cm² (mg of composition per square centimeter of coated substrate), and
wherein the soil adhering compound is substantially not transferable to a user.

2. The personal care product according to claim 1 wherein R₁, R₃, R₄ and R₅ are independently selected from the group consisting of C₁₂-C₂₂ alkyl, C₁₂-C₂₂ alkenyl, C₁₂-C₂₂ hydroxyalkyl or C₁₂-C₂₂ alkoxyl group.

3. The personal care product according to claim 1 wherein A is selected from a polymer or copolymer of ethylene oxide, propylene oxide, butylene oxide wherein said polymer or said copolymer comprises from 70 to 190 monomers.

4. The personal care product according to claim 1 wherein A is a polymer or a copolymer of ethylene oxide, propylene oxide, butylene oxide wherein said polymer or said copolymer comprises from 2 to 200 monomers and wherein R₂ is a -O-CO-R₃ wherein R₃ is a C₁₂-C₂₂ hydrocarbyl or C₁₂-C₂₂ substituted hydrocarbyl group.

5. The personal care product according to claim 1 wherein R₁ is a C₁₇ linear alkyl group and R₂ is a -O-CO-R₃ wherein R₃ is a C₁₇ linear alkyl group and A is a polymer of ethylene oxide or a polymer of propylene oxide comprising from 2 to 200 monomers.

6. The personal care product according to any of the preceding claims wherein the substrate further comprises an emollient selected from the group consisting of petroleum-based emollient, polyethylene glycol, polyethylene glycol derivatives and combination thereof.

7. The personal care product according to any of the preceding claims wherein the composition further comprise an immobilizing agent selected from the group consisting of fatty alcohols, fatty acids, waxes, ethoxylates of fatty acids, fatty alcohols and combination thereof.

8. The personal care product according to any of the preceding claims wherein the substrate comprises a nonwoven web.

9. A method for imparting adherence for body exudates to a personal care product wherein the personal care product is selected from a diaper, a feminine hygiene product, an adult incontinence product, comprising a substrate, said method comprising the step of applying to said substrate of said personal care product at least a soil adhering compound imparting adherence for exudates to said substrate, or a composition comprising at least the soil adhering compound in an amount ranging from 10% to 99%, said compound being a solid non-ionic compound of formula:
R₁-CO-[A]-R₂
wherein R₁ is a C₁₂-C₂₂ hydrocarbyl or C₁₂-C₂₂ substituted hydrocarbyl group;
wherein R₂ is selected from:
(a) -O-CO-R₃ wherein R₃ is a C₁₂-C₂₂ hydrocarbyl or C₁₂-C₂₂ substituted hydrocarbyl group; or
(b) 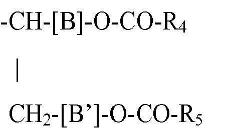
wherein R₄ is a C₁₂-C₂₂ hydrocarbyl or C₁₂-C₂₂ substituted hydrocarbyl group;
wherein R₅ is a C₁₂-C₂₂ hydrocarbyl or C₁₂-C₂₂ substituted hydrocarbyl group;
wherein A, B and B' are independently selected from:
a polymer or copolymer of ethylene oxide, propylene oxide, butylene oxide, hydroxypropylene oxide wherein said polymer or said copolymer comprises from 2 to 200 monomers,
wherein the soil adhering compound or the composition comprising the soil adhering compound is applied to the substrate in an amount ranging from 0.05 mg/cm² to 6.4 mg/cm² (mg of composition per square centimeter of coated substrate) and,
wherein the soil adhering compound is substantially not transferable to a user.

## Patentansprüche

1. Körperpflegeprodukt, wobei das Körperpflegeprodukt ausgewählt ist aus einer Windel, einem Damenhygieneprodukt und einem Erwachseneninkontinenzprodukt, wobei das Körperpflegeprodukt ein Substrat umfasst, das mindestens eine Schmutzanhaftungsverbindung umfasst, die ein Anhaften von Ausscheidungen an dem Substrat vermittelt, oder eine Zusammensetzung, die mindestens die Schmutzanhaftungsverbindung in einer Menge im Bereich von 10 % bis 99 % umfasst, wobei die Verbindung eine feste nicht ionische Verbindung der folgenden Formel ist:
R₁-CO-[A]-R₂
wobei R₁ ein C₁₂-C₂₂-Hydrocarbyl oder eine substituierte C₁₂-C₂₂-Hydrocarbylgruppe ist;
wobei R₂ ausgewählt ist aus:
(a) -O-CO-R₃, wobei R₃ ein C₁₂-C₂₂-Hydrocarbyl oder eine substituierte C₁₂-C₂₂-Hydrocarbylgruppe ist; oder
(b) 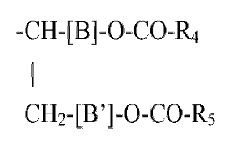
wobei R₄ ein C₁₂-C₂₂-Hydrocarbyl oder eine substituierte C₁₂-C₂₂-Hydrocarbylgruppe ist;
wobei R₅ ein C₁₂-C₂₂-ein Hydrocarbyl oder eine substituierte C₁₂-C₂₂-Hydrocarbylgruppe ist;
wobei A, B und B' unabhängig ausgewählt sind aus:
einem Polymer oder Copolymer von Ethylenoxid, Propylenoxid, Butylenoxid, Hydroxypropylenoxid, wobei das Polymer oder das Copolymer von 2 bis 200 Monomere umfasst,
wobei die Schmutzanhaftungsverbindung oder die die Schmutzanhaftungsverbindung umfassende Zusammensetzung auf das Substrat in einer Menge von 0,05 mg/cm² bis 6,4 mg/cm² (mg der Zusammensetzung pro Quadratzentimeter des beschichteten Substrats) aufgetragen wird, und
wobei die Schmutzanhaftungsverbindung im Wesentlichen nicht auf einen Benutzer übertragbar ist.

2. Körperpflegeprodukt nach Anspruch 1, wobei R₁, R₃, R₄ und R₅ unabhängig voneinander ausgewählt sind aus der Gruppe bestehend aus C₁₂-C₂₂-Alkyl-, C₁₂-C₂₂-Alkenyl-, C₁₂-C₂₂-Hydroxyalkyl- oder C₁₂-C₂₂-Alkoxylgruppe.

3. Körperpflegeprodukt nach Anspruch 1, wobei A ausgewählt ist aus einem Polymer oder Copolymer von Ethylenoxid, Propylenoxid, Butylenoxid, wobei das Polymer oder das Copolymer von 70 bis 190 Monomere umfasst.

4. Körperpflegeprodukt nach Anspruch 1, wobei A ist ein Polymer oder ein Copolymer von Ethylenoxid, Propylenoxid, Butylenoxid ist, wobei das Polymer oder das Copolymer von 2 bis 200 Monomere umfasst und wobei R₂ ein -O-CO-R₃ ist, wobei R₃ ein C₁₂-C₂₂-Hydrocarbyl oder eine substituierte C₁₂-C₂₂-Hydrocarbylgruppe ist.

5. Körperpflegeprodukt nach Anspruch 1, wobei R₁ eine lineare C₁₇-Alkylgruppe ist und R₂ ein -O-CO-R₃ ist, wobei R₃ eine lineare C₁₇-Alkylgruppe ist und A ein Polymer von Ethylenoxid oder ein Polymer von Propylenoxid ist und von 2 bis 200 Monomere umfasst.

6. Körperpflegeprodukt nach einem der vorstehenden Ansprüche, wobei das Substrat ferner ein Erweichungsmittel umfasst, das ausgewählt ist aus der Gruppe bestehend aus Erweichungsmittel auf Erdölbasis, Polyethylenglycol, Polyethylenglycolderivaten und Kombinationen davon.

7. Körperpflegeprodukt nach einem der vorstehenden Ansprüche, wobei die Zusammensetzung ferner ein Immobilisierungsmittel ausgewählt aus der Gruppe bestehend aus Fettalkoholen, Fettsäuren, Wachsen, Ethoxylaten von Fettsäuren, Fettalkoholen und Kombinationen davon umfasst.

8. Körperpflegeprodukt nach einem der vorstehenden Ansprüche, wobei das Substrat eine Vliesbahn umfasst.

9. Verfahren zum Verleihen einer Anhaftung für Körperausscheidungen an ein Körperpflegeprodukt, wobei das Körperpflegeprodukt ausgewählt ist aus einer Windel, einem Damenhygieneprodukt und einem Erwachseneninkontinenzprodukt, umfassend ein Substrat, wobei das Verfahren den Schritt des Aufbringens von mindestens einer Schmutzanhaftungsverbindung, die eine Anhaftung für Ausscheidungen an dem Substrat vermittelt, oder einer Zusammensetzung, die die Schmutzanhaftungsverbindung in einer Menge im Bereich von 10 % zu 99 % umfasst, auf das Substrat des Körperpflegeprodukts umfasst, wobei die Verbindung eine feste nicht ionische Verbindung der folgenden Formel ist:
R₁-CO-[A]-R₂
wobei R₁ ein C₁₂-C₂₂-Hydrocarbyl oder eine substituierte C₁₂-C₂₂-Hydrocarbylgruppe ist;
wobei R₂ ausgewählt ist aus:
(a) -O-CO-R₃, wobei R₃ ein C₁₂-C₂₂-Hydrocarbyl oder eine substituierte C₁₂-C₂₂-Hydrocarbylgruppe ist; oder
(b) 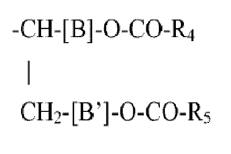
wobei R₄ ein C₁₂-C₂₂-Hydrocarbyl oder eine substituierte C₁₂-C₂₂-Hydrocarbylgruppe ist;
wobei R₅ ein C₁₂-C₂₂-ein Hydrocarbyl oder eine substituierte C₁₂-C₂₂-Hydrocarbylgruppe ist;
wobei A, B und B' unabhängig ausgewählt sind aus:
einem Polymer oder Copolymer von Ethylenoxid, Propylenoxid, Butylenoxid, Hydroxypropylenoxid, wobei das Polymer oder das Copolymer von 2 bis 200 Monomere umfasst,
wobei die Schmutzanhaftungsverbindung oder die die Schmutzanhaftungsverbindung umfassende Zusammensetzung auf das Substrat in einer Menge von 0,05 mg/cm² bis 6,4 mg/cm² (mg der Zusammensetzung pro Quadratzentimeter des beschichteten Substrats) aufgetragen wird, und
wobei die Schmutzanhaftungsverbindung im Wesentlichen nicht auf einen Benutzer übertragbar ist.

## Revendications

1. Produit de soins personnels dans lequel le produit personnel est choisi parmi une couche, un produit d'hygiène féminine et un produit pour l'incontinence chez l'adulte, le produit de soins personnels comprenant un substrat comprenant au moins un substrat d'adhérence de salissures communiquant une adhérence pour des exsudats audit substrat, ou une composition comprenant au moins le substrat d'adhérence de salissures en une quantité allant de 10 % à 99 %, ledit composé étant un composé non ionique solide de formule :
R₁-CO-[A]-R₂
dans lequel R₁ est un groupe hydrocarbyle en C₁₂ à C₂₂ ou hydrocarbyle substitué en C₁₂ à C₂₂ ;
dans lequel R₂ est choisi parmi :
(a) -O-CO-R₃ dans lequel R₃ est un groupe hydrocarbyle en C₁₂ à C₂₂ ou hydrocarbyle substitué en C₁₂ à C₂₂ ; ou
(b) 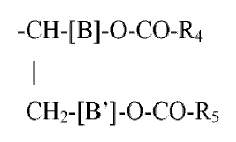
dans lequel R₄ est un groupe hydrocarbyle en C₁₂ à C₂₂ ou hydrocarbyle substitué en C₁₂ à C₂₂ ;
dans lequel R₅ est un groupe hydrocarbyle en C₁₂ à C₂₂ ou hydrocarbyle substitué en C₁₂ à C₂₂ ;
dans lequel A, B et B' sont indépendamment choisis parmi :
un polymère ou copolymère d'oxyde d'éthylène, d'oxyde de propylène, d'oxyde de butylène, d'oxyde d'hydroxypropylène dans lequel ledit polymère ou ledit copolymère comprend de 2 à 200 monomères,
dans lequel le substrat d'adhérence de salissures ou la composition comprenant le substrat d'adhérence de salissures est appliqué au substrat en une quantité allant de 0,05 mg/cm² à 6,4 mg/cm² (mg de composition par centimètre carré de substrat revêtu), et
dans lequel le substrat d'adhérence de salissures est essentiellement non transférable à un utilisateur.

2. Produit de soins personnels selon la revendication 1 dans lequel R₁, R₃, R₄ et R₅ sont indépendamment choisis dans le groupe constitué d'un groupe alkyle en C₁₂ à C₂₂, alcényle en C₁₂ à C₂₂, hydroxyalkyle en C₁₂ à C₂₂ ou alcoxyle en C₁₂ à C₂₂.

3. Produit de soins personnels selon la revendication 1 dans lequel A est choisi parmi un polymère ou copolymère d'oxyde d'éthylène, d'oxyde de propylène, d'oxyde de butylène dans lequel ledit polymère ou ledit copolymère comprend de 70 à 190 monomères.

4. Produit de soins personnels selon la revendication 1 dans lequel A est un polymère ou un copolymère d'oxyde d'éthylène, d'oxyde de propylène, d'oxyde de butylène dans lequel ledit polymère ou ledit copolymère comprend de 2 à 200 monomères et dans lequel R₂ est un -O-CO-R₃ dans lequel R₃ est un groupe hydrocarbyle en C₁₂ à C₂₂ ou hydrocarbyle substitué en C₁₂ à C₂₂.

5. Produit de soins personnels selon la revendication 1 dans lequel R₁ est un groupe alkyle linéaire en C₁₇ et R₂ est un -O-CO-R₃ dans lequel R₃ est un groupe alkyle linéaire en C₁₇ et A est un polymère d'oxyde d'éthylène ou un polymère d'oxyde de propylène comprenant de 2 à 200 monomères.

6. Produit de soins personnels selon l'une quelconque des revendications précédentes dans lequel le substrat comprend en outre un émollient choisi dans le groupe constitué d'émollient à base de pétrole, polyéthylène glycol, dérivés de polyéthylène glycol et une combinaison de ceux-ci.

7. Produit de soins personnels selon l'une quelconque des revendications précédentes dans lequel la composition comprend en outre un agent immobilisant choisi dans le groupe constitué d'alcools gras, acides gras, cires, éthoxylates d'acides gras, alcools gras et une combinaison de ceux-ci.

8. Produit de soins personnels selon l'une quelconque des revendications précédentes dans lequel le substrat comprend une nappe non tissée.

9. Procédé pour communiquer une adhérence pour des exsudats corporels à un produit de soins personnels dans lequel le produit personnel est choisi parmi une couche, un produit d'hygiène féminine, un produit pour l'incontinence chez l'adulte, comprenant un substrat, ledit procédé comprenant l'étape consistant à appliquer audit substrat dudit produit de soins personnels au moins un substrat d'adhérence de salissures communiquant une adhérence pour des exsudats audit substrat, ou une composition comprenant au moins le substrat d'adhérence de salissures en une quantité allant de 10 % à 99 %, ledit composé étant un composé non ionique solide de formule :
R₁-CO-[A]-R₂
dans lequel R₁ est un groupe hydrocarbyle en C₁₂ à C₂₂ ou hydrocarbyle substitué en C₁₂ à C₂₂ ;
dans lequel R₂ est choisi parmi :
(a) -O-CO-R₃ dans lequel R₃ est un groupe hydrocarbyle en C₁₂ à C₂₂ ou hydrocarbyle substitué en C₁₂ à C₂₂ ; ou
(b) 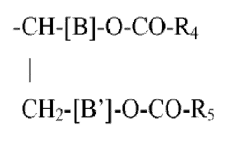
dans lequel R₄ est un groupe hydrocarbyle en C₁₂ à C₂₂ ou hydrocarbyle substitué en C₁₂ à C₂₂ ;
dans lequel R₅ est un groupe hydrocarbyle en C₁₂ à C₂₂ ou hydrocarbyle substitué en C₁₂ à C₂₂ ;
dans lequel A, B et B' sont indépendamment choisis parmi :
un polymère ou copolymère d'oxyde d'éthylène, d'oxyde de propylène, d'oxyde de butylène, d'oxyde d'hydroxypropylène dans lequel ledit polymère ou ledit copolymère comprend de 2 à 200 monomères,
dans lequel le substrat d'adhérence de salissures ou la composition comprenant le substrat d'adhérence de salissures est appliqué au substrat en une quantité allant de 0,05 mg/cm² à 6,4 mg/cm² (mg de composition par centimètre carré de substrat revêtu) et,
dans lequel le substrat d'adhérence de salissures est essentiellement non transférable à un utilisateur.
